(19) Europäisches Patentamt
European Patent Office
Office européen des brevets

(11) Veröffentlichungsnummer : **0 051 260**
**B1**

(12) # EUROPÄISCHE PATENTSCHRIFT

(45) Veröffentlichungstag der Patentschrift : **05.09.84**

(51) Int. Cl.³ : **C 07 D307/93**, A 61 K 7/46, C 11 B 9/00

(21) Anmeldenummer : **81109075.2**

(22) Anmeldetag : **28.10.81**

(54) **Methyl-4-oxatricyclo(5.2.1.02,6)-dec-8-en-3-on (I) und Methyl-4-oxatricyclo(5.2.1.02,6)-decan-3-on (II), deren Herstellung und deren Verwendung als Riechstoffe.**

(30) Priorität : **03.11.80 DE 3041251**

(43) Veröffentlichungstag der Anmeldung : **12.05.82 Patentblatt 82/19**

(45) Bekanntmachung des Hinweises auf die Patenterteilung : **05.09.84 Patentblatt 84/36**

(84) Benannte Vertragsstaaten : **AT BE CH DE FR GB IT LI NL**

(56) Entgegenhaltungen :
**DE-A- 2 517 620**
**DE-A- 2 826 302**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Upadek, Horst, Dr.**
**Kempenweg 18 A**
**D-4006 Erkrath (DE)**
Erfinder : **Bruns, Klaus, Dr.**
**Notburgaweg 6**
**D-4150 Krefeld-Traar (DE)**
Erfinder : **Conrad, Jens, Dr.**
**Dürerweg 15**
**D-4010 Hilden (DE)**

**EP 0 051 260 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es wurde gefunden, daß Methyl-4-oxatricyclo [5.2.1.$0^{2,6}$]-dec.-8-en-3-on (I) und Methyl-4-oxatricyclo [5.2.1.0.$^{2,6}$]-decan-3-on (II) der folgenden Formeln

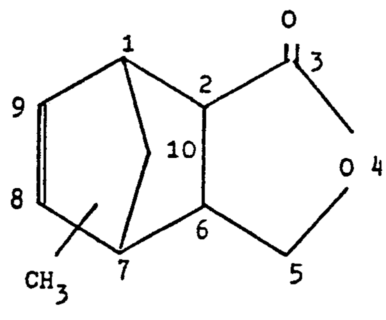

(I)

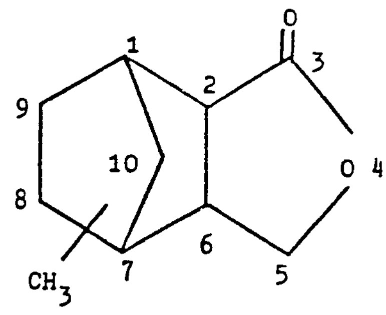

(II)

in denen die Methylgruppe die Positionen 1, 7, 8, 9, oder 10 einnimmt, und die in Form ihrer Isomerengemische vorliegen, wertvolle neue Riechstoffe darstellen, die insbesondere für neuartige Riechstoffkompositionen geeignet sind.

Aus DE-A 2 826 302 sind tricyclische Derivate von Norbornan und deren Verwendung in Riechstoffkompositionen bekannt, die einen Lactonring mit 6 Atomen am Norbornansystem anelliert enthalten, wobei der Norbornanring keine Methylgruppe beträgt. Diese Verbindungen unterscheiden sich von den erfindungsgemäß beanspruchten Verbindungen nicht nur durch abweichende Partialstrukturen, sondern auch durch wesentliche stereochemische Faktoren, die sich entscheidend auf die Geruchsqualitäten auswirken. So werden die Verbindungen der DE-A-2 826 302 als sehr natürlich, grün, frisch, pflanzig, leicht fettig, gegebenenfalls an Tonkabohnen, medizinische Auszüge erinnernd beschrieben, die zum Verbessern, Verstärken und Modifizieren der geruchstragenden Eigenschaften von Parfümkompositionen dienen, während die erfindungsgemäß beanspruchten Verbindungen intensive Cumarin- und Heuwiesen-Noten bzw. Walnußduft-Noten besitzen und damit Parfümkompositionen mit charakteristischen eigenständigen Geruchsnoten ermöglichen.

Die Herstellung der erfindungsgemäßen neuen Verbindungen erfolgt nach an sich bekannten Syntheseverfahren der organischen Chemie. Als Ausgangsmaterial für die Herstellung dienen Methylcyclopentadien in der Form der dimeren Verbindung und Maleinsäureanhydrid. Durch Umsetzung im Druckgefäß bei ca. 200 °C bildet sich zunächst das monomere Methylcyclopentadien. An dieses « in situ » gebildete Dien addiert sich das Maleinsäureanhydrid im Sinne einer Diels-Adler-Reaktion unter Bildung des Addukts Methylbicyclo [2.2.1]-hept-5-en-2.3-dicarbonsäureanhydrid (III), welches auch unter der Bezeichnung « Methyl Nadio Anhydrid » als Epoxidhärter bekannt ist. Dieses Anhydrid wird mit einem komplexen Metallhydrid, vorteilhaft mit Natriumborhydrid, in Isopropanol als Lösungsmittel reduziert, wobei sich ein Gemisch der isomeren Methyl-4-oxatricyclo [5.2.1.$0^{2,6}$]-dec-8-en-3-one (IV-VIII) bildet.

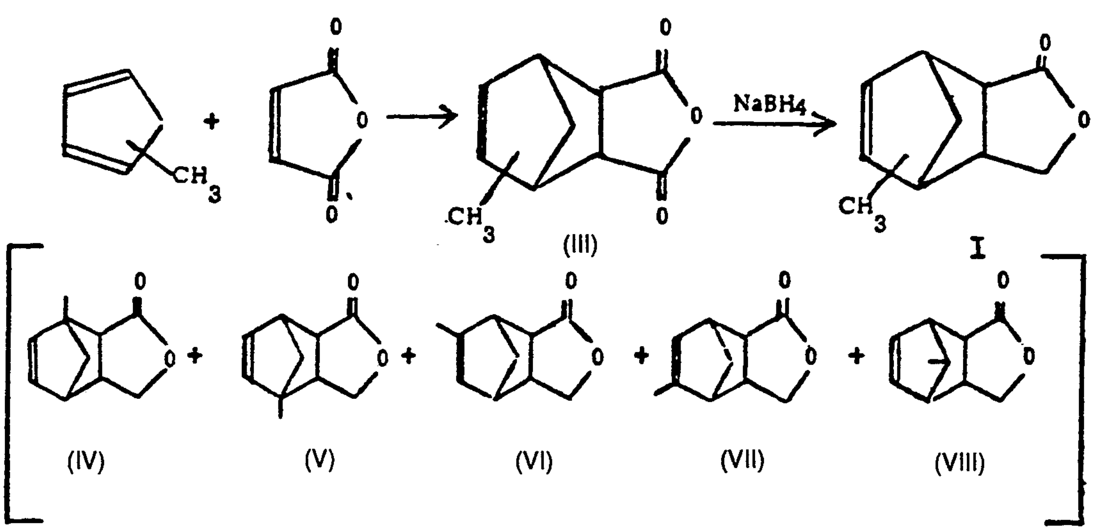


(III) I

(IV) (V) (VI) (VII) (VIII)

Dieses Lactongemisch entspricht der Formel I und enthält aufgrund einer Protonenresonanz-Analyse überwiegend die Isomeren IV-VII, das Isomere VIII nur in untergeordneter Menge. Dieses Isomerengemisch kann ohne weitere Auftrennung als Riechstoff verwendet werden.

Durch Hydrieren des ungesättigten Lactongemisches I in Gegenwart von z. B. Raney-Nickel als Katalysator wird das entsprechende Isomerengemisch der gesättigten Methyl-4-oxatricyclo [5.2.1.0$^{2,6}$]-decan-3-one (II) erhalten,

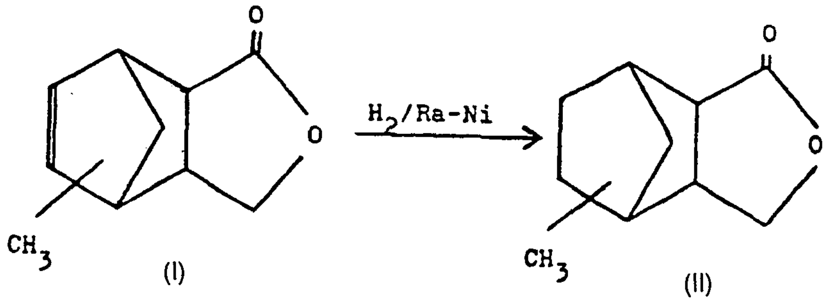


(I) (II)

welches ebenfalls ohne weitere Auftrennung als Riechstoff verwendet werden kann.

Während das Methyl-4-oxatricyclo [5.2.1.0$^{2,6}$]-dec-8-en-3-on eine intensive Cumarin- und Heuwiesen Duftnote aufweist und als Cumarin-Ersatzprodukt verwendet werden kann, zeigt das gesättigte Methyl-4-oxatricyclo [5.2.1.0$^{2,6}$]-decan-3-on eine schwach cumarinähnliche, deutliche Walnuß-Duftnote. Die neuen Riechstoffe zeichnen sich durch eine sehr gute Kombinationsfähigkeit mit anderen, natürlichen und synthetischen Riechstoffen zu neuen, interessanten Duftkompositionen aus. Sie lassen sich mit anderen Riechstoffen in verschiedenen Mengenverhältnissen mischen. Im Allgemeinen wird ihr Anteil in Riechstoffkompositionen im Bereich von 1 bis 50 Gewichtsprozent, bezogen auf die gesamte Komposition, liegen. Solche Riechstoffkompositionen können zur Parfümierung von kosmetischen Präparaten wie Duftwässern, Haarwässern, Aerosolpräparaten, Desodorantien, Cremes, Lotionen, Badepräparaten, Shampoos, Toilettenseifen sowie auch in der Extrait-Parfumerie verwendet werden. Sie können aber auch zur Geruchsverbesserung technischer Produkte, z. B. von Wasch- und Reinigungsmitteln, Wäscheweichspülmitteln, Textilbehandlungsmitteln usw. eingesetzt werden. Zur Parfümierung der verschiedenen Produkte werden diese Kompositionen im allgemeinen in Konzentrationen von 0,05 bis 2,0 Gewichtsprozent, bezogen auf das gesamte Produkt, zugesetzt.

Die nachfolgend aufgeführten Beispiele sollen den Erfindungsgegenstand näher erläutern, ohne ihn jedoch hierauf zu beschränken.

## Beispiele

### Beispiel 1

Herstellung von Methyl-4-oxatricyclo [5.2.1.0$^{2,6}$]-dec-8-en-3-on (I)

a) 49,1 g (0,5 Mol) Maleinsäureanhydrid und 40,1 g (0,25 Mol) Methylcyclopentadien-Dimeres wurden unter Zusatz von 0,5 g Hydrochinon und 100 ml Toluol 2 Stunden bei 200 °C im Autoklaven erhitzt. Durch Vakuumdestillation wurden bei 109-115 °C/0,13 mbar 40,7 g Methylbicyclo [2.2.1]-hept-5-en-2,3-dicarbonsäureanhydrid erhalten.

b) 40,7 g (0,23 Mol) Anhydrid wurden mit 11,3 g (0,3 Mol) Natriumborhydrid in 260 ml Isopropanol 7 Stunden bei 75 °C gerührt. Nach Entfernung der Hauptmenge an Isopropanol wurden 90 g konzentrierte Salzsäure, verdünnt mit 200 ml Wasser, zugesetzt. Es wurde 2 Stunden bei Raumtemperatur gerührt und anschließend mit Ether mehrfach extrahiert. Die vereinigten organischen Phasen wurden mit verdünnter Sodalösung und Wasser neutral gewaschen, eingeengt und im Vakuum destilliert. Bei 84-89 °C/0,13 mbar wurden 18 g Methyl-4-oxatricyclo [5.2.1.0$^{2,6}$]-dec-8-en-3-on isoliert.

$n_D^{20}$ = 1,507 2

IR (Film) : 3 050 $cm^{-1}$ (C=C—H), 1630 $cm^{-1}$ (C=C)
1 765 $cm^{-1}$ (C=0,5-Ring Lacton), 1 380 $cm^{-1}$ ($CH_3$).

Der Geruch wird durch eine kräftige Cumarin- Heuwiese-Note charakterisiert.

### Beispiel 2

Herstellung von Methyl-4-oxatricyclo [5.2.1.0$^{2,6}$]-decan-3-on (II)

3,5 g (0,02 Mol) Methyl-4-oxatricyclo [5.2.1.0$^{2,6}$]-dec-8-en-3-on wurden in 50 ml Methanol über 0,35 g Raney-Nickel in 4 Stunden bei 120 °C und 200 bar Wasserstoff-Druck hydriert. Nach Filtration und Entfernung des Lösungsmittels wurden 3,4 g Lacton II erhalten.

Durch Kugelrohr-Destillation wurde eine reine Probe von 1,9 g isoliert.

Kp. 124 °C/0,3 mbar

$n_D^{20}$ = 1,498 6

Ir (Film) : 1 765 $cm^{-1}$ (C = 0,5-Ring-Lacton),
1 380 $cm^{-1}$ ($CH_3$)

Der Geruch wird durch eine deutliche Walnuß-Note und schwache Cumarin-Note gekennzeichnet.

## Beispiel 3

Heuwiese-Komplex für Badeformulierungen

| | |
|---|---|
| Methyl-4-oxatricyclo [5.2.1.$0^{2,6}$] dec-8-en-3-on | 140 Gewichtsteile |
| Linalool | 170 Gewichtsteile |
| Lavendelöl | 130 Gewichtsteile |
| Cumarin | 100 Gewichtsteile |
| Ethyllinalool | 100 Gewichtsteile |
| Benzylacetat | 60 Gewichtsteile |
| Benzophenon | 50 Gewichtsteile |
| Methylacetophenon | 40 Gewichtsteile |
| Geranium abs. | 30 Gewichtsteile |
| Bergamottöl | 30 Gewichtsteile |
| Lavendel abs. | 20 Gewichtsteile |
| Concrete Sauge Sclarée | 20 Gewichtsteile |
| Nerolidol | 20 Gewichtsteile |
| Cedrylacetat | 15 Gewichtsteile |
| Tonka abs. | 15 Gewichtsteile |
| Hydroxicitronellal | 10 Gewichtsteile |
| Jasmin kstl. | 10 Gewichtsteile |
| Linalylformiat | 10 Gewichtsteile |
| Foin abs. | 10 Gewichtsteile |
| Patchouliöl | 10 Gewichtsteile |
| cis-Hexenylbenzoat | 5 Gewichtsteile |
| cis-Hexenol 10 % i. DEP | 5 Gewichtsteile |
| | 1 000 Gewichtsteile |

**Ansprüche**

1. Methyl-4-oxatricyclo [5.2.1.$0^{2,6}$]-dec-8-en-3-on der Formel I und Methyl-4-oxatricyclo [5.2.1.$0^{2,6}$]-decan-3-on der Formel II

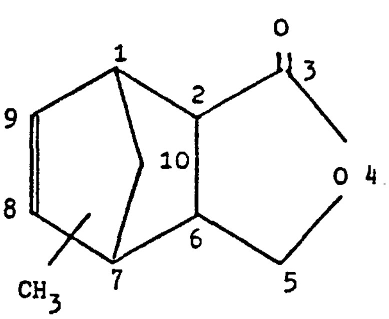

(I)

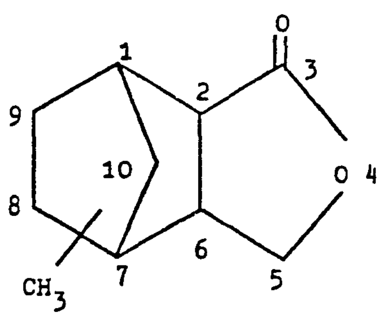

(II)

in Form der Isomerengemische, in welchen die Methylgruppe die Positionen 1, 7, 8, 9 und 10 einnimmt.

2. Verfahren zur Herstellung von Methyl-4-oxatricyclo [5.2.1.$0^{2,6}$]-dec-8-en-3-on der Formel I durch Reduktion von Methylbicyclo [2.2.1]-hept-5-en-2.3-dicarbonsäureanhydrid mit komplexen Metallhydriden.

3. Verfahren zur Herstellung von Methyl-4-oxatricyclo [5.2.1.$0^{2,6}$]-decan-3-on der Formel II durch katalytische Hydrierung des Methyl-4-oxatricyclo [5.2.1.$0^{2,6}$]-dec-8-en-3-on der Formel I.

4. Verwendung von Methyl-4-oxatricyclo [5.2.1.$0^{2,6}$]-dec-8-en-3-on und Methyl-4-oxatricyclo [5.2.1.$0^{2,6}$]-decan-3-on nach Anspruch 1 als Riechstoffe.

5. Riechstoffkompositionen dadurch gekennzeichnet, daß sie Methyl-4-oxatricyclo [5.2.1.$0^{2,6}$]-dec-8-en-3-on oder Methyl-4-oxatricyclo [5.2.1.$0^{2,6}$]-decan-3-on in einer Menge von 1 bis 50 Gewichtsprozent, bezogen auf die gesamte Komposition, enthalten.

**Claims**

1. Methyl-4-oxatricyclo [5.2.1.0$^{2,6}$]-dec-8-en-3-one corresponding to formula I and methyl-4-oxatricyclo [5.2.1.0$^{2,6}$]-decan-3-one corresponding to formula II

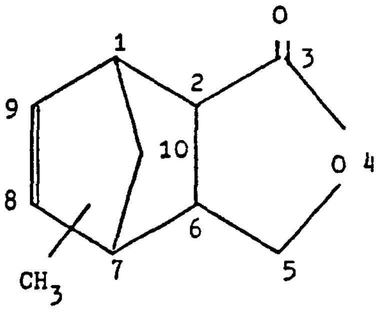

(I)

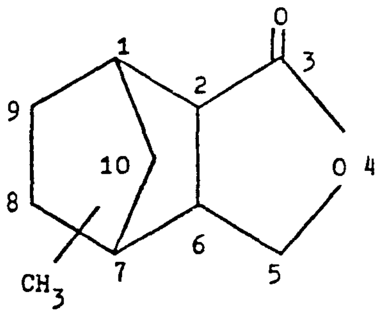

(II)

in the form of the isomer mixtures in which the methyl group occupies the 1, 7, 8, 9 and 10 positions.

2. A process for producing the methyl-4-oxatricyclo [5.2.1.0$^{2,6}$]-dec-8-en-3-one corresponding to formula I by reducing methylbicyclo [2.2.1]-hept-5-ene-2.3-dicarboxylic acid anhydride with complex metal hydrides.

3. A process for producing the methyl-4-oxatricyclo [5.2.1.0$^{2,6}$]-decan-3-one corresponding to formula II by catalytically hydrogenating the methyl-4-oxatricyclo [5.2.1.0$^{2,6}$]-dec-8-en-3-one corresponding to formula I.

4. The use of the methyl-4-oxatricyclo [5.2.1.0$^{2,6}$]-dec-8-en-3-one and the methyl-4-oxatricyclo [5.2.1.0$^{2,6}$]-decan-3-one claimed in Claim 1 as perfumes.

5. Perfume compositions, characterized in that they contain methyl-4-oxatricyclo [5.2.1.0$^{2,6}$]-dec-8-en-3-one or methyl-4-oxatricyclo [5.2.1.0$^{2,6}$]-decan-3-one in a quantity of from 1 to 50 % by weight, based on the composition as a whole.

**Revendications**

1. Méthyl-4-oxatricyclo [5.2.1.0$^{2,6}$]-déc-8-ène-3-one de formule I et méthyl-4-oxatricyclo [5.2.1.0$^{2,6}$]-décane-3-one de formule II :

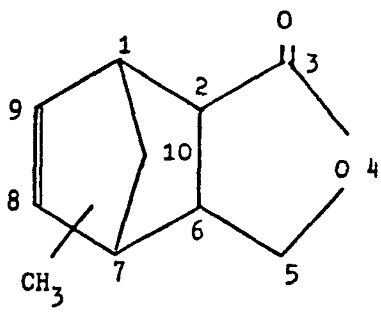

(I)

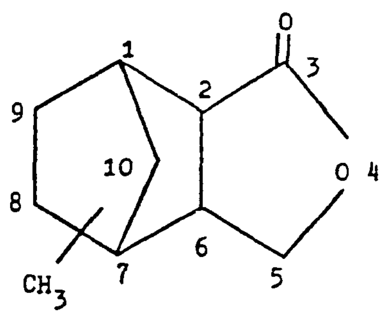

(II)

sous forme des mélanges isomères dans lesquels le groupe méthyle occupe les positions 1, 7, 8, 9 et 10.

2. Procédé de fabrication de méthyl-4-oxatricyclo [5.2.1.0$^{2,6}$]-déc-8-ène-3-one de formule I par réduction de l'anhydride méthylbicyclo [2.2.1.] hept-5-ène-2,3-dicarboxylique avec des hydrures métalliques complexes.

3. Procédé de fabrication de méthyl-4-oxatricyclo [5.2.1.0$^{2,6}$]-décane-3-one de formule II par hydrogénation catalytique de la méthyl-4-oxatricyclo [5.2.1.0$^{2,6}$] déc-8-ène-3-one de formule I.

4. Utilisation de la méthyl-4-oxatricyclo [5.2.1.0$^{2,6}$]-déc-8-ène-3-one et de la méthyl-4-oxatricyclo [5.2.1.0$^{2,6}$]-décane-3-one selon la revendication 1 comme substances odorantes.

5. Compositions de substances odorantes, caractérisées en ce qu'elles contiennent de la méthyl-4-oxatricyclo [5.2.1.0$^{2,6}$]-déc-8-ène-3-one ou de la méthyl-4-oxatricyclo [5.2.1.0$^{2,6}$]-décane-3-one en une quantité de 1 à 50 % en poids par rapport à la composition totale.